# EUROPÄISCHE PATENTANMELDUNG

(11) **EP 1 161 968 A1**
(43) Veröffentlichungstag der Anmeldung: **12.12.2001**
(21) Anmeldenummer: 00112091.4
(22) Anmeldetag: 05.06.2000
(51) Int. Cl.: A63B 21/28, A63B 21/008

(54) **Tauziehvorrichtung**

(71) Anmelder: Pixelpark AG, 10245 Berlin (DE)
(72) Erfinder: Gorny, Alexander, 50668 Köln (DE); Ludwigs, Stefan, 50677 Köln (DE)
(74) Vertreter: Glawe, Delfs, Moll & Partner

(57) **Zusammenfassung**

Gegenstand der Erfindung ist eine Tauziehvorrichtung, mit einem Seil (7) mit einem maschinenseitigem und einem freien Seilende, einer Einrichtung (2) zum Beaufschlagen des maschinenseitigen Seilendes mit einer Zugkraft und zum Verändern der Länge des freien Seilendes. Erfindungsgemäß ist vorgesehen, daß diese Vorrichtung folgende Merkmale aufweist:
- einen Kraftsensor,
- eine Längenmesseinrichtung,
- eine Schnittstelle zum Übertragen von Meßdaten,
- eine Einrichting zum Vergleichen von Meßdaten von anderen Tauziehvorrichtungen, die zum Einstellen der Zugkraft und der Seillänge dienen.

Erfindungsgemäße Tauziehvorrichtungen können über Datenleitungen so miteinander verbunden werden, daß ein telematisches Tauziehen gegen eine räumlich entfernte Partei möglich ist.

## Beschreibung

Die Erfindung betrifft eine Tauziehvorrichtung mit einem Seil, das ein aus der Vorrichtung ragendes freies Ende und ein mit der Vorrichtung verbundenes maschinenseitiges Ende aufweist, einer Einrichtung zum Beaufschlagen des maschinenseitigen Seilendes mit einer Zugkraft und zum Verändern der Länge des aus der Vorrichtung ragenden freien Seilendes.

Der Erfindung liegt die Aufgabe zugrunde, eine solche Tauziehvorrichtung zu schaffen, die eine realistische Simulation eines Tauziehens mit einem menschlichen Gegenüber ermöglicht.

Die Erfindung löst diese Aufgabe durch folgende Merkmale der Tauziehvorrichtung:
- einen Sensor zum Messen der auf das freie Seilende wirkenden Zugkraft,
- eine Einrichtung zum Messen der Länge des aus der Vorrichtung ragenden freien Seilendes,
- eine Schnittstelle, die zum Übertragen der Meßdaten an wenigstens eine weitere Tauziehvorrichtung und zum Empfang entsprechender Meßdaten von anderen Tauziehvorrichtungen ausgebildet ist,
- eine Einrichtung zum Vergleichen gemessener und von weiteren Tauziehvorrichtungen empfangener Zugkraft- und Seillängendaten und zum Betätigen der Einrichtung zum Beaufschlagen des maschinenseitigen Seilendes mit einer Zugkraft und zum Verändern der Länge des aus der Vorrichtung ragenden freien Seilendes in Abhängigkeit von den Ergebnissen des vorgenommenen Vergleichs.

Zunächst seien einige im Rahmen der Erfindung verwendete Begriffe erläutert.

Der Begriff "Seil" ist funktionell zu verstehen und umfaßt jegliche Einrichtung, die von einem Menschen mit einer Zugkraft beaufschlagt werden kann. In der Regel wird es sich um ein übliches flexibles Seil handeln, u.U. kann ein solches Seil jedoch auch als starre Zugstange oder dergleichen ausgebildet sein. Das aus der Vorrichtung ragende freie Ende des Seils dient der Handhabung durch den oder die tauziehenden Personen. Das maschinenseitige Ende wird direkt oder ggf. über Umlenkeinrichtungen, Flaschenzüge oder dergleichen mit einer maschinenseitig erzeugten Zugkraft beaufschlagt. Die Länge des aus der Vorrichtung ragenden freien Seilendes ist veränderlich, sie hängt in unten noch näher beschriebener Weise vom Stand des simulierten Tauziehens ab.

"Aus der Vorrichtung ragen" bedeutet, daß das entsprechende Seilende für einen Zugriff für tauziehende Personen zur Verfügung steht. Aus Sicherheitsgründen wird ein solcher Zugriff nicht über die gesamte Länge des freien Endes möglich oder erlaubt sein, damit ziehende Personen nicht in die Maschine und/oder auf Seilumlenkeinrichtungen oder dergleichen der Maschine hineingezogen werden können.

Erfindungsgemäß ist vorgesehen sowohl ein Kraftsensor zum Messen der auf das freie Seilende aufgebrachten Zugkraft als auch ein Weglängensensor zum Messen der Länge des aus der Vorrichtung ragenden freien Seilendes. Diese beiden Sensoren messen sowohl die Kraft als auch die Position des Taues bzw. Seils.

Ferner ist eine Schnittstelle vorgesehen, die zum Übertragen der Meßdaten dieser Sensoren an wenigstens eine weitere Tauziehvorrichtung und zum Empfang entsprechender Meßdaten von anderen Tauziehvorrichtungen ausgebildet ist.

Erfindungsgemäß können somit zwei oder mehr Tauziehvorrichtungen mittels Datenfernübertragung so miteinander verbunden werden, daß zwei oder mehr Personen auch räumlich getrennt voneinander ein "telematisches" Tauziehen gegeneinander durchführen können. Die Datenfernübertragung zwischen den Schnittstellen kann mittels dem Fachmann geläufiger Datenprotokolle erfolgen, bspw. mittels des TCP/IP-Protokolls über das Internet, über sonstige Datenfernleitungen, über das Telefonnetz, über Funk oder Satellitenverbindungen oder dergleichen. Es ist dann eine Einrichtung vorgesehen, die die gemessenen eigenen Kraft- und Weglängendaten und die von einer oder mehreren weiteren Tauziehvorrichtungen empfangenen entsprechenden Daten miteinander vergleicht und in Abhängigkeit vom Ergebnis des Vergleichs die Stellglieder zur maschinenseitigen Zugkraftbeauschlagung des Seils und zum Verändern der freien Seillänge betätigt. Dadurch vermittelt das jeweilige Stellglied der Tauziehvorrichtung der am freien Seilende ziehenden Person das Gefühl, gegen ein menschliches Gegenüber zu ziehen, da die entsprechenden Kraft- und Wegdaten tatsächlich von einem menschlichen Gegenüber in einer zweiten Tauziehvorrichtung stammen. Werden mehrere Tauziehvorrichtungen miteinander verbunden, kann bspw. jede Tauziehvorrichtung mit dem Durchschnitt der Kraft und/oder Wegdaten der Summe der anderen Tauziehvorrichtungen beaufschlagt werden.

Die Einrichtung zum Vergleichen gemessener und von weiteren Tauziehvorrichtungen empfangener Zugkraft- und Seillängendaten ist bevorzugt in jede Tauziehvorrichtung (bspw. in den dort sowieso vorhandenen Rechner) integriert, dies ist jedoch nicht zwingend erforderlich. Es kann auch entweder eine einzige Tauziehvorrichtung diese Einrichtung aufweisen und dann mittels Datenfernübertragung die Stellglieder (Einrichtung zum Beaufschlagen des maschinenseitigen Seilendes mit einer Zugkraft und zum Verändern der Länge des aus der Vorrichtung rangenden freien Seilendes) alle anderen Tauziehvorrichtungen unmittelbar steuern. Ferner kann diese Vergleichseinrichtung auch getrennt von sämtlichen Tauziehvorrichtungen als separate Einrichtung vorgesehen sein, bspw. kann sie einem in das Datennetzwerk der Tauziehvorrichtungen eingebundenen Moderator zugeordnet sein, der die Tauziehwettbewerbe leitet und ggf. steuernd eingreifen kann.

Im Rahmen der Erfindung ist es möglich, die Tauziehvorrichtung nicht mit einer physischen zweiten Tauziehvorrichtung zu verbinden, sondern mit einer bspw. durch Software simulierten zweiten Tauziehvorrichtung (virtuelles Tauziehen oder Tauziehen gegen einen virtuellen Partner). Der Gegenstand des Anspruchs 1 erfordert lediglich, daß die Schnittstelle sich zum Übertragen entsprechender Daten an andere Tauziehvorrichtungen eignet, nicht jedoch, daß solche weiteren Tauziehvorrichtungen tatsächlich angeschlossen sind. Hier kann statt dessen auch die genannte virtuelle Tauziehvorrichtung als Gegenüber angeschlossen werden.

Wenn im Rahmen der Erfindung von Datenfernübertragung die Rede ist, bedeutet dies nicht, daß eine bestimmte räumliche Mindestdistanz überwunden werden muß. Es wird lediglich verlangt, daß Daten über die genannten Schnittstellen in einem Format übertragen werden, das sich grundsätzlich auch zum Übertragen über größere Distanzen hinweg eignet.

Die Einrichtung zum Beaufschlagen des maschinenseitigen Seilendes mit einer Zugkraft und zum Verändern der Länge des aus der Vorrichtung ragenden freien Seilendes (das Stellglied oder die Stelleinrichtung für das Seil) weist vorzugsweise einen pneumatischen oder hydraulischen Zylinder auf. Ein hydraulischer Zylinder mit unveränderlichem Querschnitt über den gesamten Verfahrweg ist bevorzugt, da dann der Verfahrweg (die Längenänderung des Seils) unmittelbar proportional der dem Zylinder zugeführten Menge an Hydraulikflüssigkeit ist. Das Stellglied kann einfachwirkend ausgebildet sein und ist dementsprechend nur in der Lage, das freie Seilende in die Maschine hinauszuziehen. Alternativ kann das Stellglied doppelwirkend ausgebildet und damit auch zum Herausfahren des Seils aus der Maschine vorgesehen sein. Die doppeltwirkende Ausbildung erleichtert bspw. nach einem Wettbewerb das Zurückfahren des Seils in die Ausgangsstellung. Dabei kann vorgesehen sein, daß das Stellglied das Seil nur dann aktiv in Richtung auf eine Vergrößerung der freien Seillänge verfährt, wenn von außen eine gewisse Mindestzugkraft auf dieses Seilende aufgebracht wird, damit sich nicht loses Seil in der Maschine verheddert. Die aktive Unterstützung des Herausziehens von Seil durch einen doppeltwirkenden Zylinder kann auch dann sinnvoll sein, wenn in der Tauziehvorrichtung größere Reibungskräfte auftreten und bspw. auch Kinder zur Betätigung der Vorrichtung in der Lage sein sollen.

Die Ansteuerung des Hydraulikzylinders erfolgt in dem Fachmann geläufiger Weise durch eine Ölpumpe und ein oder mehrere vorzugsweise stufenlos ansteuerbare Servoventile oder Proportionalventile.

Das maschinenseitige Seilende kann ortsfest mit der Tauziehvorrichtung verbunden sein und das Stellglied (bspw. der hydraulische oder pneumatische Zylinder) auf eine Seilumlenkrolle wirken. "Ortsfest" bedeutet in diesem Fall eine feste Verbindung mit dem Chassis, Gehäuse oder dergleichen der Tauziehvorrichtung. Mit der Seilumlenkrolle erhält man eine Kraft- und Weguntersetzung. Mit einer einfachen Seilumlenkrolle kann bspw. der Hub eines Hydraulikzylinders verdoppelt werden, d.h. das freie Seilende kann um den doppelten Hub des Zylinders verfahren werden. Bei Bedarf sind entsprechend größere Untersetzungen mit einem Flaschenzug möglich. Im Rahmen der Erfindung kann das Seil bei Bedarf in verschiedenen Längenabschnitten aus unterschiedlichen Materialien bestehen, bspw. kann das auf der Vorrichtung ragende freie Seilende ein übliches geflochtenes Tau aus Kunstfasern, Hanf oder dergleichen sein; der innerhalb der Tauziehvorrichtung verlaufende Seilteil kann bspw. als Stahlseil ausgebildet sein.

Der Sensor zum Messen der auf das freie Seilende wirkenden Zugkraft ist bevorzugt ortsfest im Bereich des maschinenseitigen Seilendes angeordnet. Der Sensor kann bspw. das Widerlager für das maschinenseitige Seilende bilden, er kann alternativ innerhalb desjenigen Seilabschnitts angeordnet sein, der zwischen maschinenseitigem Seilende und (erster) Seilumlenkrolle verläuft. Sofern eine solche Seilumlenkrolle, ein Flaschenzug oder eine sonstige Kraftuntersetzung vorgesehen ist, muß der Meßwert des Sensors ggf. mit einem entsprechenden Korrekturfaktor beaufschlagt werden.

Die Einrichtung bzw. der Sensor zum Messen der Länge des aus der Vorrichtung ragenden freien Seilendes kann bevorzugt als Wegaufnehmer ausgebildet sein, der den Hub des pneumatischen bzw. Hydraulikzylinders mißt. Bei einer Untersetzung der Bewegung des Hydraulikzylinders ist auch hier wieder ggf. ein Korrekturfaktor vorzusehen.

Die erfindungsgemäße Vorrichtung kann zusätzlich ein Display (bspw. ein Monitor oder dergleichen) und/oder eine optische Aufzeichnungseinrichtung (bspw. eine Videokamera) sowie eine Schnittstelle zum Austauschen von Bilddaten mit weiteren Tauziehvorrichtungen und/oder anderen Bilddatenverarbeitungsstationen aufweisen. Beim telematischen Tauziehen gegen eine räumlich entfernte Partei können auf diese Weise bspw. Bilddaten des jeweiligen Gegenübers ausgetauscht werden. Display und Kamera sind bevorzugt in Blickrichtung einer ziehenden Person (vorzugsweise etwa in Augenhöhe) angeordnet. In das Display können außer Videobilder eines telematischen Gegenübers andere optisch aufbereitete Daten eingespeist werden, bspw. Daten der Kraft- und Seilweglängensensoren, Zwischenergebnisse eines Wettbewerbs, die verbleibende Zeit oder dergleichen. Die genannten weiteren Bilddatenverarbeitungsstationen können bspw. Videobilder des Tauziehens aufbereiten und für dritte Zuschauer verfügbar machen, ein Moderator kann über solche Stationen eine optische Aufbereitung von Daten vornehmen und weitere Bilder (bspw. aus dem Umfeld eines solchen Wettbewerbes) für die Parteien einspeisen.

In gleicher Weise wie für Bilddaten können Aufzeichnungs-, Wiedergabe- und Schnittstelleneinrichtungen für Tondaten vorgesehen sein.

Im Rahmen der Erfindung ist es bevorzugt, wenn sämtliche auszutauschende Daten (Meßdaten der Sensoren, ggf. Bilddaten und Tondaten) über einheitliche Schnittstellen und ein einheitliches Datenprotokoll miteinander ausgetauscht werden. Dies setzt voraus, daß eine entsprechend breitbandige Verbindung zur Verfügung steht. Unter Umständen kann es sinnvoll sein, bspw. die Sensordaten über das TCP/IP-Protokoll (Internet) auszutauschen und in Ermangelung einer hinreichend breitbandigen Internetverbindung insbesondere für Bilddaten ein anderes Protokoll vorzusehen, das mit hinreichend breitbandigen Verbindungen arbeitet, bspw. der Austausch über ISDN-Datenleitungen, Funk- bzw. Satellitenverbindungen oder dergleichen.

Bevorzugt ist eine Sicherheitseinrichtung vorgesehen, die die Verkürzung des aus der Vorrichtung ragenden freien Seilendes unterbricht (vorzugsweise die Tauziehvorrichtung vollständig abschaltet), wenn eine an dem freien Ende ziehende Person einen vorgegebenen Sicherheitsabstand zum Eintrittsbereich des Seils in die Vorrichtung unterscheidet. Der Begriff "Eintrittsbereich" bezeichnet entweder eine entsprechende Gehäuseöffnung für das Seil oder ggf. eine Umlenkeinrichtung für das Seil. Diese Sicherheitseinrichtung kann bspw. eine Lichtschranke und/oder einen Trittsensor (bspw. eine mit einem Trittsensor ausgerüstete Fußmatte) aufweisen.

Gegenstand der Erfindung ist ferner eine Anordnung von wenigstens zwei mittels Datenleitungen verbundenen Tauziehvorrichtungen. Diese Anordnung weist einen Zugkraftregelkreis und einen Seillängenregelkreis auf. Diese Regelkreise bewirken, daß bei jeder Tauziehvorrichtung die von der Vorrichtung auf das freie Seilende ausgeübte Zugkraft sowie die konkrete Verfahrstellung des Seils stets Zugkraft und den entsprechenden Weg des "telematischen Gegenübers" repräsentieren. Werden mehrere Tauziehvorrichtungen zu einer erfindungsgemäßen Anordnung verbunden, kann bspw. vorgesehen sein, daß jede Vorrichtung gegen die Kraftdurchschnittswerte aller anderen Vorrichtungen zieht.

Der Zugkraftregelkreis und der Seillängenregelkreis müssen so miteinander abgestimmt werden, daß zum einen die Zugkräfte an den freien Seilenden zweier zusammengeschalteter Maschinen stets übereinstimmen (Kraft gleich Gegenkraft) und zum anderen der Verfahrweg des freien Seilendes einer Maschine dem negativen Verfahrweg des Seilendes der anderen Maschine entspricht.

Angemerkt sei, daß ggf. die gemessenen Zugkräfte vor dem Einspeisen in den Regelkreis mit einem vorgegebenen Faktor beaufschlagt werden können, so daß auch Parteien sehr unterschiedlicher Stärke gegeneinander Tauziehen können, bspw. Kinder gegen Erwachsene, eine Einzelperson gegen eine Mannschaft oder dergleichen. Der gleiche Faktor findet dann natürlich auch Anwendung bei der Betätigung des Stellgliedes (bspw. des Hydraulikzylinders) der zugehörigen Tauziehvorrichtung. Dieser Faktor findet Anwendung nur im Zugkraftregelkreis, nicht jedoch im Seillängenregelkreis.

Erfindungsgemäß kann vorgesehen sein, daß der Zugkraftregelkreis Priorität gegenüber dem Seillängenregelkreis aufweist, so daß zu jedem Zeitpunkt die grundlegende Bedingung des Tauziehens, nämlich die Übereinstimmung der jeweiligen Zugkräfte (ggf. mit dem genannten Faktor beaufschlagt), gewährleistet ist.

Im Seillängenregelkreis kann man eine Tauziehvorrichtung fest als sogenannten Master vorgeben, dessen Wegaufnehmer das Stellglied für die Seillänge des telematischen Gegenübers steuert. Bei der Ausbildung des Stellgliedes als hydraulischer Zylinder ist jedoch zu berücksichtigen, daß das Verfahren des hydraulischen Zylinders mit Pumpendruck zur Verkürzung des freien Seilendes in der Regel schneller vonstatten gehen kann als das Verfahren in die umgekehrte Richtung, da das Ölvolumen bei einem doppeltwirkend beaufschlagten Zylinder auf der Kolbenunterseite größer ist als auf der Kolbenoberseite, auf der die Kolbenstange ein gewisses Volumen einnimmt. Erfindungsgemäß ist es daher bevorzugt, wenn in dem Seillängenregelkreis die Funktion des sogenannten Masters wechselt und stets derjenigen Tauziehvorrichtung zugeordnet wird, die gerade gewissermaßen die Oberhand hat, auf deren freies Seilende also die höchste Zugkraft ausgeübt wird. Es kann dann vorgesehen sein, daß die Druckbeaufschlagung des zugehörigen Hydraulikzylinders vollständig eingestellt und das Ausströmventil vollständig geöffnet wird, so daß das freie Seilende dieser Station gegen den Strömungswiderstand des aus dem Zylinder ausströmenden Hydrauliköls herausgezogen werden kann. Diejenige Station mit der höchsten Zugkraft bekommt gewissermaßen stets sofort Seil, wie es ja auch beim echten Tauziehen der Fall ist. Der Sensor zum Messen der freien Seillänge dieser Vorrichtung mit der höchsten Zugkraft gibt dann im Seillängenregelkreis den Weg vor, um die das freie Seilende der Tauziehvorrichtung des telematischen Gegenübers verkürzt werden muß. Bei dieser Station wird dann der Hydraulikzylinder entsprechend druckbeaufschlagt, dazu muß das Einströmventil des entsprechenden Zylinders üblicherweise nicht vollständig geöffnet werden. Somit ist an dem Einströmventil dieses Zylinders stets eine Regelungsreserve vorhanden.

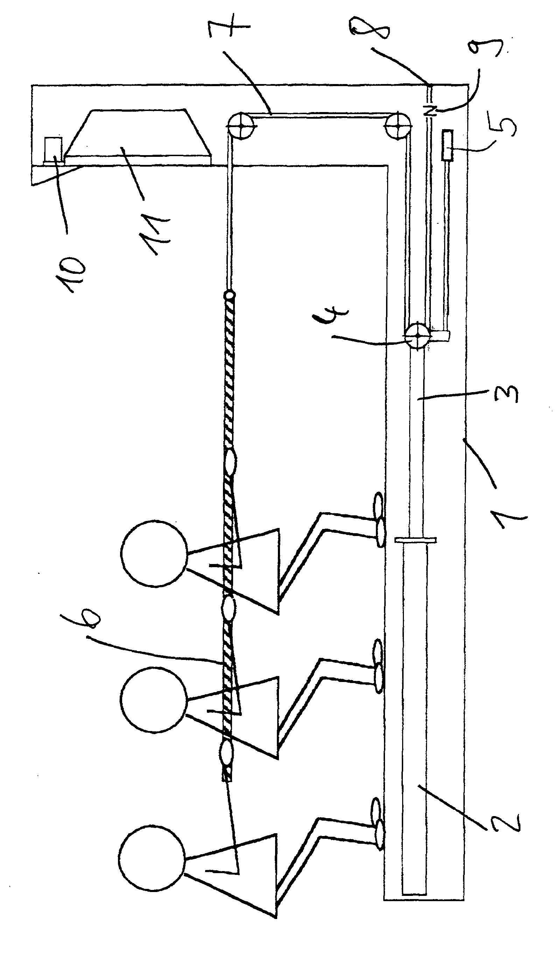

Ein Ausführungsbeispiel einer erfindungsgemäßen Tauziehvorrichtung wird im folgenden anhand der Zeichnung erläutert.

In einem Gehäuse 1 ist ein Hydraulikzylinder 2 angeordnet, der einen Kolben 3 betätigt. Dieser Zylinder kann bspw. einen maximalen Hub von 1,5 m sowie eine maximale Zugkraft von 2 t aufweisen. Der Zylinder wird in der Zeichnung nicht dargestellter, jedoch dem Fachmann geläufiger Weise über ein Hydraulikaggregat und Servoventile mit Hydraulikflüssigkeit beaufschlagt. Der Kolben 3 des Hydraulikzylinders 2 betätigt eine Seilumlenkrolle 4, an der ein Wegaufnehmer 5 zur Erfassung der Länge des freien Seilendes 6 ist.

Das Seil 7 ist mit seinem maschinenseitigen Ende bei 8 mit dem Gehäuse 1 der Tauziehvorrichtung verbunden, die auf das freie Seilende 6 wirkende Zugkraft wird mit einem Kraftsensor 9 gemessen. Eine Videokamera 10 sowie ein Monitor 11 dienen zur Aufzeichnung bzw. Wiedergabe der Bilddaten.

Die Funktion der einzelnen Elemente der erfindungsgemäßen Tauziehvorrichtung sowie ihr Zusammenwirken mit einem telematischen bzw. virtuellen Gegenüber ist vorstehend bereits ausführlich beschrieben worden.

## Patentansprüche

1. Tauziehvorrichtung, mit einem Seil (7), das ein aus der Vorrichtung ragendes freies Ende (6) und ein mit der Vorrichtung verbundenes maschinenseitiges Ende aufweist, einer Einrichtung (2) zum Beaufschlagen des maschinenseitigen Seilendes mit einer Zugkraft und zum Verändern der Länge des aus der Vorrichtung ragenden freien Seilendes, **gekennzeichnet durch** folgende Merkmale:
- einen Sensor (9) zum Messen der auf das freie Seilende (6) wirkenden Zugkraft,
- eine Einrichtung (5) zum Messen der Länge des aus der Vorrichtung ragenden freien Seilendes (6),
- eine Schnittstelle, die zum Übertragen der Meßdaten an wenigstens eine weitere Tauziehvorrichtung und zum Empfang entsprechender Meßdaten von anderen Tauziehvorrichtungen ausgebildet ist,
- eine Einrichtung zum Vergleichen gemessener und von weiteren Tauziehvorrichtungen empfangener Zugkraftund Seillängendaten und zum Betätigen der Einrichtung zum Beaufschlagen des maschinenseitigen Seilendes mit einer Zugkraft und zum Verändern der Länge des aus der Vorrichtung ragenden freien Seilendes in Abhängigkeit von den Ergebnissen des vorgenommenen Vergleichs.

2. Tauziehvorrichtung nach Anspruch 1, **dadurch gekennzeichnet, daß** die Einrichtung zum Beaufschlagen des maschinenseitigen Seilendes mit einer Zugkraft und zum Verändern der Länge des aus der Vorrichtung ragenden freien Seilendes (6) einen pneumatischen oder hydraulischen Zylinder (2) aufweisen.

3. Tauziehvorrichtung nach Anspruch 2, **dadurch gekennzeichnet, daß** das maschinenseitige Seilende ortsfest mit der Tauziehvorrichtung verbunden ist und der Zylinder (2) auf eine Seilumlenkrolle (4) wirkt.

4. Tauziehvorrichtung nach Anspruch 2, **dadurch gekennzeichnet, daß** der Sensor (9) zum Messen der auf das freie Seilende wirkenden Zugkraft ortsfest im Bereich des maschinenseitigen Seilendes angeordnet ist.

5. Tauziehvorrichtung nach einem der Ansprüche 1 bis 4, **dadurch gekennzeichnet, daß** sie zusätzlich ein Display (11) und/oder eine optische Aufzeichnungseinrichtung (10) sowie eine Schnittstelle zum Austausch von Bilddaten mit anderen Tauziehvorrichtungen und/oder anderen Bilddatenverarbeitungsstationen aufweist.

6. Tauziehvorrichtung nach einem der Ansprüche 1 bis 5, **dadurch gekennzeichnet, daß** sie zusätzlich eine akustische Wiedergabe- und/oder eine Aufzeichnungseinrichtung sowie eine Schnittstelle zum Austausch von Tondaten mit anderen Tauziehvorrichtungen und/oder anderen Tondatenverarbeitungsstationen aufweist.

7. Tauziehvorrichtung nach einem der Ansprüche 1 bis 6, **dadurch gekennzeichnet, daß** sie eine Sicherheitseinrichtung aufweist, die die Verkürzung des aus der Vorrichtung ragendes freien Seilendes unterbricht, wenn eine an dem freien Ende ziehende Person einen vorgegebenen Sicherheitsabstand zum Eintrittsbereich des Seils in die Vorrichtung unterschreitet.

8. Tauziehvorrichtung nach Anspruch 7, **dadurch gekennzeichnet, daß** die Sicherheitseinrichtung eine Lichtschranke und/oder einen Trittsensor aufweist.

9. Anordnung von wenigstens zwei mittels Datenleitungen verbundenen Tauziehvorrichtungen nach einem der Ansprüche 1 bis 6, **dadurch gekennzeichnet, daß** sie einen Zugkraftregelkreis und einen Seillängenregelkreis aufweist.

10. Anordnung nach Anspruch 9, **dadurch gekennzeichnet, daß** der Zugkraftregelkreis Priorität gegenüber dem Seillängenregelkreis aufweist.

11. Anordnung nach Anspruch 9 oder 10, **dadurch gekennzeichnet, daß** die gemessenen Zugkräfte einer oder mehrerer Tauziehvorrichtungen vor dem Einspeisen in den Regelkreis mit einem vorgegebenen Faktor beaufschlagt werden.

12. Anordnung nach Anspruch 10 oder 11, **dadurch gekennzeichnet, daß** die Tauziehvorrichtung mit der höchsten gemessenen Zugkraft die Funktion des Masters im Seillängenregelkreis übernimmt.
